**Europäisches Patentamt**

**European Patent Office**

**Office européen des brevets**

(19)

(11) Publication number: **0 271 455**
A1

(12)

# EUROPEAN PATENT APPLICATION

(21) Application number: **87830409.6**

(22) Date of filing: **17.11.87**

(51) Int. Cl.⁴: **A 61 K 7/16**
A 61 K 7/18

---

(30) Priority: **18.11.86 IT 2237486**

(43) Date of publication of application:
**15.06.88 Bulletin 88/24**

(84) Designated Contracting States:
**CH DE ES FR GB LI SE**

(71) Applicant: **S. APOLLONIA CORPORATION S.r.L.**
**Via Canonica, 47**
**Milano (IT)**

(72) Inventor: **Ponchio, Giuliano**
**Via XX Settembre 39**
**Verbania Novara (IT)**

(74) Representative: **Pizzoli, Pasquale et al**
**c/o Società Italiana Brevetti Via G. Carducci 8**
**I-20123 Milano (IT)**

---

(54) **Fluoride containing collutory for caries prevention.**

(57) A fluoride-containing collutory to be used as a prophylactic aid for the prevention of caries is characterized by a prolonged dwelling time of the fluoride ions in the oral cavity, which is achieved by means of adding thickeners into the collutory composition, carboxymethyl cellulose being the preferred thickener.

EP 0 271 455 A1

Bundesdruckerei Berlin

**Description**

"Fluoride containing collutory for caries prevention"

The present invention relates to a fluoride -containing collutory to be employed as a prophylactic aid against caries which is characterized by a prolonged dwelling time of fluoride ions in the oral cavity after expulsion of the collutory.

It is known that the arising of the dental caries during infancy and the development age has a direct relation with the deficiency of fluoride ion in the body. It has already been proposed to make up for the lack of said ion, which is normally assimilated by the organism through food and expecially through drinking water, by adding fluoride ions to drinking waters lacking or devoid thereof to bring them to a concentration up to 1 ppm, that is 1 mg/l. Said proposal has aroused many perplexities due to the technical difficulty partly related to the irrational design of some water distribution networks and partly to problems concerning the personnel in charge of operations and monitoring. To overcome said difficulties, it has also been proposed to use collutories, that is aqueous or aquo-alcoholic solutions containing fluoride ions to be used as mouth-washes lasting a few tens of seconds. The pharmacological action of said collutories is rather limited because the dwelling time of fluoride ions in the oral cavity after ejecting the collutory is rather short, whilst the biochemical process of fluoride ions assimilation through the walls of gums and the surface of teeths is rather slow.

It is the object of the present invention to extend the dwelling time of the fluoride ions in the oral cavity after ejecting the collutory thus affording a remarkable increase in the amount of absorbed fluoride ions. The above-mentioned objects is achieved by means of the present invention by providing for the modification of the chemical composition of the known collutories so as to increase their viscosity, for example by making use of thickeners in the composition of the collutory. A particular suitable thickener is carboxymethyl-cellulose.

The amount of thickener in the collutory is to be chosen so as to provide for maximum retention of fluoride ions in the oral cavity without altering at the same time the characteristics of the collutory in such a way as to make it disagreeable. It is evident that the amount that must be introduced into the composition of the collutory is to be determined for every kind of thickener by means of suitable prior determination of the fluorides. To this purpose the potentiometric method can be suitably applied by using the fluoride-ion-selective crystal membrane electrode Orion 94-09 with single-junction combination reference electrode Orion 90-01-00 connected to an Orion 701/A digital potentiometer.

In the following examples there are illustrated the analytical procedures for the determination of the optimum amount of thickener that is to be introduced in the collutory composition, the preparation of a typical collutory and the determination of the retention time of fluoride ions in the oral cavity.

Esample 1

Five aqueous solutions containing 0.5 g/l of sodium fluoride (RP Carlo Erba), equivalent to a concentration of 23 mg/l of fluoride ion also containing carboxymethyl-cellulose (CMC) in increasing amounts from 1 to 3 g/l of CMC were prepared. To all of the five solutions there was then added an equivalent volume of a suitable means for adjusting the ionic strength (TISAB II) as per Orion's directions on the 90-01-00 electrode that was used for the potentiometric determination with the above-mentioned procedure. The potential measurements were performed under mild stirring by means of a magnetic stirrer reading the related mV after a few minutes of apparatus stabilization time. A calibration curve was prepared on semilogarithmic paper by applying the same analytical conditions to four fluoride ions standards with tenfold increasing concentration from 0.1 to 100 ppm F-.

A one minute mouth-wash was performed with 10 ml each of the five above-mentioned solutions, followed by ejection. After waiting 10 minutes without swallowing or otherwhise engaging the oral cavity the mouth was subjected to a 1 minute rinse with 10 ml of deionized water. The rinsing solution was subjected after the ejection from the oral cavity to a determination of the fluoride ions by means of the above-mentioned method. The results of the performed tests are shown in table 1 below.

TABLE 1          F-Concentration (mg/l)

CMC Concentration (g/l)

| | 1 | 1.5 | 2 | 2.5 | 3 |
|---|---|---|---|---|---|
| Initial solution | 23 | 23 | 23 | 23 | 23 |
| 10 min. rinse liquid | 4 | 6.2 | 7.5 | 10 | 13 |

From table 1 it is clearly evident that the thickener affects the retention of the fluoride ion in the oral cavity at a concentration higher that 1 g/l and at a level of about 2.5 g/l it ensures the retention of nearly half of the fluoride ions that had originally been introduced into the oral cavity. Concentrations higher than 2.5 g/l impart to the collutory a slippery mouth-feel that makes it disagreeable, although they provide better retention of the fluoride ion. A concentration of about 2.6 g/l can therefore be suitably selected as the best compromise between the pharmacological effect aimed at and the organoleptic qualities of the collutory.

Example 2

By means of known technical procedures, a collutory was prepared containing 0.5 g of sodium fluoride, 2.5 g of sodium carboxymethyl-cellulose thickener, 1.0 g of Parametil bacteriostatic, 0.3 g of spearmint essence, 15 mg of E 131 blue colorant to improve the organoleptic characteristics of the collutory as well as three tablets of saccharine sweetener in one liter of water. Four 10 ml samples of said collutory were prepared and administered to four subjects, two adults and two adolescents of both sexes. 10 ml of collutory were kept for 1 minute in the oral cavity of each subject before ejecting them. The oral cavity was then rinsed with 10 ml of deionized water that after expulsion was subjected to the determination of the concentration of fluoride ions by ion-specific-electrode potentiometry. After several trials, the time intervals between the ejection of the collutory and the next rinse with deionized water, on a range of 0-7 hours, were defined for determination of an exhaustion curve of the fluoride ions in the oral cavity.

The same tests were carried out by administering to the same subjects a known collutory available on the market under the trade mark "FLUORINSE" that shows the same concentration of 23 mg/l of fluoride ions but contains no carboxymethyl-cellulose nor any other thickener.

Preliminary tests had already excluded any possible interference by the various additives present in the two samples compared in the potentiometric determination carried out.

The scattering of the results obtained over the same time with the various subjects is to be regarded as acceptable because the standard deviation is almost the same through all the series of measurements. The data are in absolute micrograms of fluoride ion present in the oral cavity at the time of rinsing with deionized water. The results of the tests are shown in table 2.

TABLE 2

| Time delay between ejection and rinsing HOURS | Retained g of F- of the collutory of the invention | | | | | Retained g of F- of known commercially available collutory | | | | |
|---|---|---|---|---|---|---|---|---|---|---|
| | TESTS | | | | AVERAGE | TESTS | | | | AVERAGE |
| | A | B | C | D | | E | F | G | H | |
| 0 | 105 | 98 | 96 | 108 | 102 | 47 | 48 | 46 | 48 | 47 |
| 1 | 45 | 56 | 46 | 48 | 49 | 19 | 18 | 20 | 24 | 20 |
| 2.5 | 29 | 37 | 26 | 31 | 31 | 16 | 11 | 15 | 15 | 14 |
| 4.5 | 17 | 18 | 24 | 22 | 20 | 12 | 10 | 11 | 12 | 11 |
| 7 | 9 | 10 | 9 | 13 | 10 | 8 | 9 | 9 | 10 | 9 |

From table 2 it appears evident that the amount of fluoride ions remaining in the oral cavity after ejecting the collutory is relatively low, as is typical of this specific form of administration. However, the presence of the thickener succeeds in doubling the amount of fluoride ions that are retained in the oral cavity raising it from about 50 μg to more than 100 μg when the oral cavity is rinsed immediately after ejecting the collutory.

From the table it is also evident that the depletion curve of the fluoride ions in the oral cavity is asymptotic and reaches a kind of biochemical equilibrium around the value of about 10 mg of fluoride ions after 7 hours dwelling time in the oral cavity. It can be finally noticed that a remarkable drop of the amount of fluoride ions retained, and presumably a fixation thereof, occurs during the first 2-3 hours.

All the tests carried out have demonstrated that the presence of the thickener in the collutory causes the fluoride ions to dwell longer on the surface of the oral cavity. Their progressive decrease is likely to mean that the fluoride ions take part in the biochemical fixation reaction on the teeth.

The collutory composition that has been described in example 2 is only to be considered as illustrative and not limiting the invention.

**Claims**

1. A fluoride-containing collutory for caries prevention containing fluoride ions, a bacteriostatic, an aromatic essence and other typical components of collutories, characterized in that it also comprises a thickener.

2. A collutory as claimed in claim 1, characterized in that the thickener is carboxymethyl cellulose.

3. A collutory as claimed in claim 1 or 2, characterized in that the thickener is added in an amount of from 1.5 to 3 g/l.

4. A collutory as claimed in claim 3, characterized in that the thickener is added in an amount of from 2.3 to 2.7 g/l.